# EUROPÄISCHE PATENTSCHRIFT

(11) **EP 2 446 842 B1**
(45) Veröffentlichungstag und Bekanntmachung des Hinweises auf die Patenterteilung: **02.12.2015**
(21) Anmeldenummer: 10013960.9
(22) Anmeldetag: 26.10.2010
(51) Int. Cl.: A61B 17/70, A61B 17/68, A61B 17/86, A61B 17/72, A61B 17/00

(54) **Vorrichtung zum Stabilisieren einer Wirbelsäule**
Device for stabilising a spine
Dispositif de stabilisation d'une colonne vertébrale

(43) Veröffentlichungstag der Anmeldung: 02.05.2012
(73) Patentinhaber: Röbling, Christian, 79104 Freiburg (DE); Rieger, Claudia, 06108 Halle/Saale (DE); Hedayat, Farman, 50931 Köln (DE)
(72) Erfinder: Röbling, Christian, 79104 Freiburg (DE); Rieger, Claudia, 06108 Halle/Saale (DE); Hedayat, Farman, 50931 Köln (DE)
(74) Vertreter: Westphal, Mussgnug & Partner Patentanwälte mbB

(56) Entgegenhaltungen:
- US-A1- 2002 198 527
- US-A1- 2005 143 735
- US-A1- 2006 052 788
- US-A1- 2008 221 623
- US-A1- 2009 131 992
- US-A1- 2010 168 751

## Beschreibung

Die Erfindung betrifft eine Vorrichtung zum Stabilisieren einer Wirbelsäule.

Bekannt ist es, eine Wirbelsäule dadurch zu stabilisieren, dass benachbarte Wirbelkörper mittels eines Stabsystems miteinander verbunden werden. Dazu werden in die benachbarten Wirbelkörper vier Pedikelschrauben quer zur Längsachse der Wirbelsäule eingesetzt und jeweils zwei Pedikelschrauben von benachbarten Wirbelkörpern mittels eines Stabs miteinander verbunden. Eine derartige Stabilisierung erfordert einen starken Eingriff beim Patienten und zieht in der Regel ein Weichteiltrauma über eine Länge von 10 cm oder mehr mit sich.

Als Stand der Technik werden die US 2009/0131992 A1, US 2008/0221623 A1, US 2010/0168751 A1, US 2005/01A3735 A1, US 2006/0052788 A1 und US 2002/0198527 A1 genannt. Die US 2010/0168751 A1 offenbart ein Verfahren und eine Vorrichtung zur perkutanen Expansion des Spinalkanals, welche insbesondere zwei Hülsen umfasst, wobei der Abstand der beiden Hülsen durch Eindrehen einer Schraube variiert werden kann. Dazu weist die erste Hülse ein Innengewinde mit einer ersten Steigung und die zweite Hülse ein Innengewinde mit einer zweiten Steigung sowie die Schraube zwei Abschnitte mit zwei unterschiedlichen Gewindesteigungen auf.

Die Aufgabe der Erfindung besteht darin, eine Vorrichtung zum Stabilisieren einer Wirbelsäule bereitzustellen, welche mit wenigen Bauteilen eine zuverlässige Stabilisierung der Wirbelsäule bewirkt und insbesondere mit einem nur geringen Weichteiltrauma einsetzbar ist. Die Aufgabe wird erfindungsgemäß durch eine Vorrichtung zum Stabilisieren einer Wirbelsäule mit den Merkmalen des Patentanspruchs 1 oder des Patentanspruchs 7 gelöst.

Vorteilhafte Ausgestaltungen und Weiterbildungen der Erfindung sind in den Unteransprüchen angegeben.

Die erfindungsgemäße Vorrichtung zum Stabilisieren einer Wirbelsäule weist eine Hülse zum Einbringen in einen Wirbelkörper auf, welche in einem Abschnitt ein Außengewinde aufweist, sowie eine Schraube. Die Hülse kann auf einfache Art und Weise implantiert werden und kann in Kombination mit der Schraube eine Stabilisierung zweier benachbarter Wirbelkörper bewirken und insbesondere ein aufwändiges Stabsystem mit mehreren Pedikelschrauben ersetzen. Die Zahl der zu implantierenden Elemente kann somit deutlich verringert werden. Die Hülse und die darin angeordnete Schraube können von dorso-medial durch einen oberen Wirbelkörper und den daran anschließenden Zwischenwirbelraum bis in den benachbarten unteren Wirbelkörper eingeführt werden, wodurch eine zuverlässige Stabilisierung der beiden benachbarten Wirbelkörper gegeneinander erreicht werden kann, jedoch lediglich ein kleiner Zugang im Patienten vonnöten ist, insbesondere ein minimalinvasiver Zutritt ermöglicht wird.

Gemäß der Erfindung weist die Hülse ein erstes Innengewinde mit einer ersten Steigung auf, in welche die Schraube eindrehbar ist. Die Hülse dient dabei zur Stabilisierung der Schraube innerhalb des Wirbelkörpers. Mit Hilfe der Schraube wird eine zuverlässige Stabilisierung zweier benachbarter Wirbelkörper gegeneinander möglich.

Gemäß der Erfindung weist die Hülse einen vorderen Abschnitt, einen mittleren Abschnitt und einen hinteren Abschnitt auf, wobei der vordere Abschnitt das Außengewinde aufweist und in dem mittleren Abschnitt Spreizelemente angeordnet sind. Nach Einsetzen der Hülse können die Spreizelemente aufgespreizt werden, um einen der Wirbelkörper, durch welchen die Hülse geführt ist, zu stabilisieren, beispielsweise aufzurichten.

In dem mittleren Abschnitt der Hülse sind Öffnungen, insbesondere in Form von Schlitzen, angeordnet, welche insbesondere in Längsrichtung der Hülse angeordnet sind, zwischen welchen die Spreizelemente gebildet sind. Diese Ausgestaltung ermöglicht es auf konstruktiv einfache Art und Weise, die Spreizelemente in der Hülse auszubilden.

Gemäß der Erfindung weist die Hülse das erste Innengewinde in dem vorderen Abschnitt auf, wobei die Schraube einen vorderen Abschnitt mit einem ersten Außengewinde mit einer dritten Steigung und einen hinteren Abschnitt mit einem zweiten Außengewinde mit einer vierten Steigung aufweist, wobei die dritte Steigung der ersten Steigung entspricht und die vierte Steigung von der dritten Steigung verschieden ist. Die Schraube ist dabei insbesondere als Zug- oder Kompressionsschraube ausgebildet und bewirkt beim Eindrehen in die Hülse, dass der vordere Abschnitt gegen den hinteren Abschnitt gezogen wird und dadurch die Spreizelemente aufspreizen. Weiterhin werden die beiden benachbarten Wirbelkörper gegeneinander verspannt und dadurch, beispielsweise in Kombination mit einem Zwischenwirbelimplantat, zuverlässig stabilisiert.

Vorteilhafterweise sind die Spreizelemente in Umfangsrichtung der Hülse unterschiedlich ausgebildet, um gegebenenfalls der asymmetrischen Anordnung in der Hülse in dem Wirbelkörper und der Notwendigkeit, den Wirbelkörper in unterschiedlichen Richtungen unterschiedlich stark aufzurichten und zu stabilisieren, Rechnung tragen zu können.

Vorteilhafterweise weisen die Spreizelemente Sollknickstellen auf, um ein definiertes Aufspreizen der Spreizelemente der Hülse zu ermöglichen.

Vorzugsweise weist die Schraube an ihrem Schraubenschaft einen Anschlag auf, welcher insbesondere konisch ausgebildet ist, sodass das dem Kopf zugewandte Ende der Hülse an den Anschlag anschlägt und die Zugwirkung oder Kompressionswirkung der Schraube unterstützt.

Gemäß einer bevorzugten Ausführungsform der Erfindung ist die Hülse zumindest in einem mittleren Abschnitt aus einer Memory-Legierung, insbesondere aus Nitinol gefertigt. Die Memory-Legierung ist insbesondere so gewählt, dass sie bei Erreichen der Körpertemperatur ihre Form ändert. Dabei ist insbesondere vorgesehen, dass diese Formänderung ein Aufspreizen vorhandener Spreizelemente bewirkt. Dadurch kann auf eine Schraube verzichtet werden und eine Stabilisierung der beiden benachbarten Wirbelkörper und ein Aufrichten oder Stabilisieren eines der Wirbelkörper ausschließlich durch die Hülse erreicht werden.

Das Einsetzen der Hülse und gegebenenfalls einer Schraube wird dadurch vereinfacht, dass die Hülse und die Schraube kannuliert ausgebildet sind, um zunächst einen Führungsdraht einführen zu können und anschließend entlang dieses Führungsdrahts die Hülse und gegebenenfalls die Schraube einführen zu können.

Eine Alternative der Erfindung sieht vor, dass die Hülse das erste Innengewinde in einem hinteren Abschnitt aufweist und dass die Schraube einen vorderen Abschnitt mit einem ersten Außengewinde mit einer dritten Steigung und einen hinteren Abschnitt mit einem zweiten Außengewinde mit einer vierten Steigung aufweist, wobei die vierte Steigung der ersten Steigung entspricht. Auf diese Weise wird es ermöglicht, zunächst lediglich die Schraube in die Wirbelkörper einzuführen und schließlich die Hülse auf den hinteren Abschnitt der Schraube aufzudrehen.

Gemäß der Alternative ist die Schraube in einem Bereich zwischen dem vorderen Abschnitt und dem hinteren Abschnitt elastisch ausgebildet. Verbleibt dieser Bereich nach Einbringen der Schraube in die Wirbelsäule im Zwischenwirbelraum zwischen zwei benachbarten Wirbelkörpern, ist es möglich, durch diesen elastischen Bereich eine Bandscheibe zu simulieren, welche durch den vorderen Abschnitt und den hinteren Abschnitt der Schraube in den beiden Wirbelkörpern verankert ist.

Die Erfindung wird anhand der nachfolgenden Figuren ausführlich erläutert. Es zeigt
- Figur 1: eine perspektivische Ansicht eines ersten Ausführungsbeispiels einer Hülse,
- Figur 1a: eine Draufsicht auf das hintere Ende der Hülse gemäß Figur 1,
- Figur 2: die Hülse gemäß Figur 1 mit darin einzuführender Schraube,
- Figur 3: die Hülse gemäß Figur 1 mit darin eingesetzter Schraube,
- Figur 4: die Hülse gemäß Figur 1 mit daran angesetztem Eindrehinstrument,
- Figur 5: eine perspektivische Ansicht eines zweiten Ausführungsbeispiels einer Hülse,
- Figur 6: die Hülse gemäß Figur 5 in aufgespreiztem Zustand,
- Figur 7: eine Seitenansicht der Hülse gemäß Figur 5,
- Figur 8: eine Ausschnittsvergrößerung der Hülse gemäß Figur 5,
- Figur 9: eine Seitenansicht eines dritten Ausführungsbeispiels einer Hülse,
- Figur 10: die Hülse gemäß Figur 9 in teilweise geschnittenem Zustand,
- Figur 11: eine Draufsicht auf das hintere Ende der Hülse gemäß Figur 9,
- Figur 12: die Hülse gemäß Figur 9 in teilweise eingedrehtem Zustand,
- Figur 13: die Hülse gemäß Figur 9 in eingedrehtem Zustand,
- Figur 14: eine weitere Ansicht der Hülse gemäß Figur 13,
- Figur 15: einen Längsschnitt durch die Hülse gemäß Figur 9,
- Figur 16: eine schematische Darstellung der Hülse gemäß Figur 1 in in einen Wirbelkörper eingesetztem Zustand,
- Figur 17: eine schematische Darstellung der Hülse gemäß Figur 9 in in einen Wirbelkörper eingesetztem Zustand,
- Figur 18: die Hülse gemäß Figur 17 in weiter in den Wirbelkörper eingedrehtem Zustand in einer Seitenansicht,
- Figur 19: eine Frontansicht der Hülse gemäß Figur 18 in in den Wirbelkörper eingesetztem Zustand,
- Figur 20: eine Seitenansicht eines vierten Ausführungsbeispiels einer Hülse mit darin eingesetzter Schraube,
- Figur 21: die Hülse gemäß Figur 20 mit nur teilweise eingesetzter Schraube,
- Figur 22: die Hülse gemäß Figur 20 mit der Schraube gemäß Figur 20,
- Figur 23: eine schematische Darstellung der Schraube gemäß Figur 22 in in einen Wirbelkörper eingesetztem Zustand,
- Figur 24: die Schraube gemäß Figur 22, auf welche die Hülse gemäß Figur 22 aufgedreht wird,
- Figur 25: die Schraube gemäß Figur 22, auf welche die Hülse gemäß Figur 22 aufgedreht wird mit einer weiteren Position der Hülse,
- Figur 26: die Schraube gemäß Figur 22, auf welche die Hülse gemäß Figur 22 aufgedreht wird mit einer weiteren Position der Hülse und
- Figur 27a, b: die Schraube gemäß Figur 22, auf welche die Hülse gemäß Figur 22 aufgedreht wird mit einer weiteren Position der Hülse.

In den Figuren sind gleiche Teile mit gleichen Bezugsziffern bezeichnet, wobei zur besseren Übersicht nicht sämtliche Bezugsziffern in sämtlichen Figuren angegeben sind.

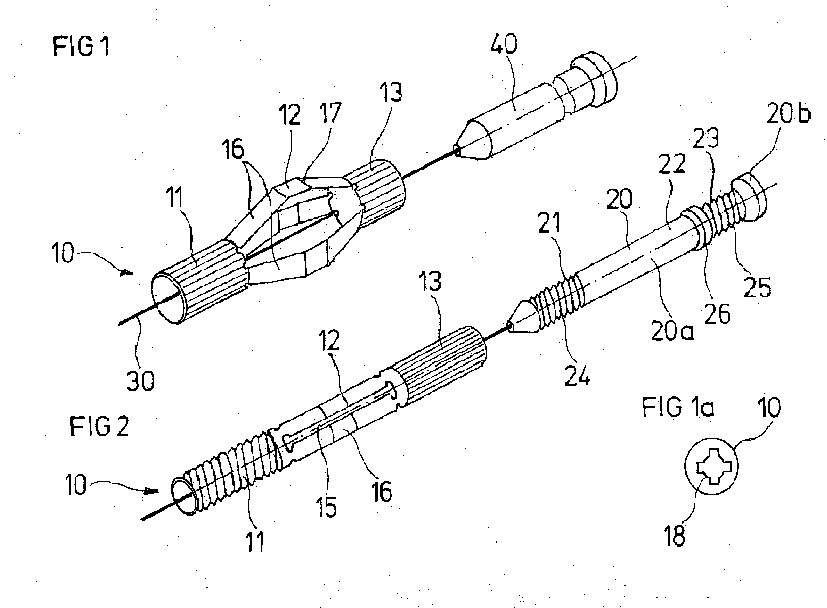

Die Figuren 1 bis 4 zeigen verschiedene Ansichten eines ersten Ausführungsbeispiels einer Hülse 10 mit einem vorderen Abschnitt 11, einem sich daran anschließenden mittleren Bereich 12 und einem sich daran anschließenden hinteren Bereich 13. Der vordere Bereich 11 trägt ein Außengewinde 14. Der hintere Bereich 13 ist auf seiner Außenseite glatt oder gegebenenfalls in Längsrichtung strukturiert ausgebildet. In dem mittleren Bereich sind in Längsrichtung der Hülse 10 mehrere Schlitze 15 angeordnet, zwischen welchen Spreizelemente 16 gebildet sind. Vorliegend sind die Schlitze 15 über den Umfang regelmäßig verteilt angeordnet. Insgesamt werden vier Spreizelemente 16 gebildet. Die Zahl der Spreizelemente 16 kann jedoch auch höher oder niedriger sein. Insbesondere können die Spreizelemente 16 auch unsymmetrisch über den Außenumfang der Hülse 10 verteilt angeordnet und ausgeformt sein.

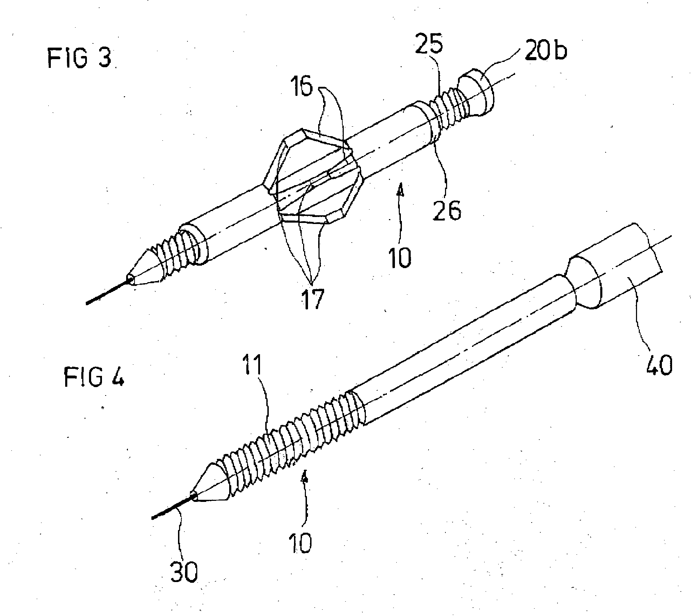

In dem vorderen Bereich 11 der Hülse 10 ist ein erstes Innengewinde angeordnet, welches eine erste Steigung aufweist. In die Hülse 10, insbesondere in das erste Innengewinde der Hülse 10, ist eine Schraube 20 eindrehbar, welche einen Schaft 20a und einen Kopf 20b aufweist. Der Schaft 20a weist einen vorderen Abschnitt 21, einen daran anschließenden mittleren Bereich 22 und einen daran anschließenden hinteren Abschnitt 23 auf, wobei sich an den hinteren Abschnitt 23 der Kopf 20b anschließt. In dem vorderen Abschnitt 21 der Schraube 20 ist ein erstes Außengewinde 24 angeordnet, während in dem hinteren Abschnitt 23 ein zweites Außengewinde 25 angeordnet ist. Das erste Außengewinde 24 weist dabei eine dritte Steigung auf, während das zweite Außengewinde 25 eine vierte Steigung aufweist. Die dritte Steigung des ersten Außengewindes 24 entspricht jedoch insbesondere der ersten Steigung des ersten Innengewindes der Hülse 10. Die dritte Steigung und die vierte Steigung sind dabei unterschiedlich gewählt, sodass die Schraube 20 als Zug- oder Kompressionsschraube wirkt. Wenn die Schraube 20 in die Hülse 10 eingedreht wird, wie insbesondere in Figur 3 ersichtlich, wird durch die unterschiedlichen Steigungen des ersten und zweiten Außengewindes 24, 25 der vordere Abschnitt 11 gegen den hinteren Abschnitt 13 gezogen, wobei sich die in der mittleren Bereich angeordneten Spreizelemente 16 nach außen radial aufspreizen. Die Spreizelemente 16 weisen dabei Sollknickstellen 17 auf, die ein definiertes Aufspreizen der Spreizelemente 16 gewährleisten sollen.

Die Hülse 10 kann auch lediglich den vorderen Abschnitt 11 mit dem Außengewinde 14 aufweisen, ohne die daran anschließenden mittleren und hinteren Bereiche 12, 13 (nicht dargestellt), um eine Stabilisierung der Schraube 20 in dem Wirbelkörper zu bewirken.

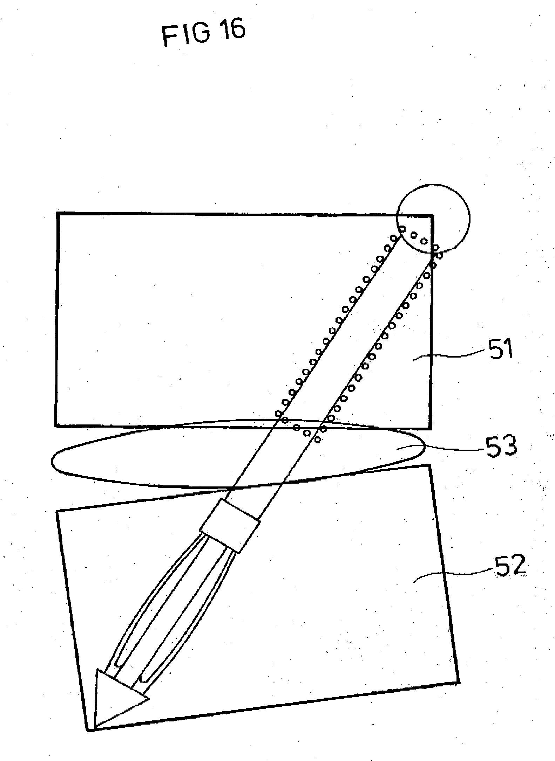

Zum Einsetzen der Hülse 10 in Wirbelkörper 51, 52 (vgl. Figur 16) wird ein Eindrehinstrument 40 verwendet. Die Hülse 10 weist dabei an ihrem einen Ende, insbesondere an dem freien Ende des hinteren Abschnitts 13, eine unrunde Kontur 18 (vgl. Fig. la) auf, in welcher eine entsprechend ausgeformte Kontur des Eindrehinstruments 40 eingreift, um die Hülse in die Wirbelkörper 51, 52 einzuschrauben. Wie anhand von Figur 16 ersichtlich, in welchen zwei benachbarte Wirbelkörper 51, 52 mit einem dazwischen liegenden Zwischenwirbelraum 53 schematisch dargestellt sind, wird die Hülse 10 durch den oberen Wirbelkörper 51 bis in den unteren Wirbelkörper 52 eingedreht, wobei die Hülse 10 den Zwischenwirbelraum 53 durchsetzt. Der mittlere Bereich 12 mit den Aufspreizelementen 16 kommt innerhalb des unteren Wirbelkörpers 52 zu liegen. Wird anschließend die Schraube 20 eingedreht, spreizen sich die Spreizelemente 16 innerhalb des Wirbelkörpers 52 auf, um diesen beispielsweise aufzurichten und zu stabilisieren.

An dem Schraubenschaft 20a, insbesondere zwischen dem mittleren Bereich 22 und dem hinteren Bereich 23 der Schraube 20, ist ein Anschlag 26 angeordnet, gegen welchen das freie Ende des hinteren Bereichs 13 der Hülse 10 beim Eindrehen der Schraube 20 anschlägt, sodass der vordere Bereich 11 der Hülse 10 gegen den hinteren Bereich 13 der Hülse 10 gezogen werden kann und sich die Spreizelemente 16 in dem mittleren Bereich 12 aufspreizen.

Die Schraube 20 ist kannuliert ausgebildet, sodass beim Implantieren zunächst ein Führungsdraht 30 eingebracht werden kann, über welchen anschließend die Hülse 10 und zuletzt die Schraube 20 eingeführt werden kann.

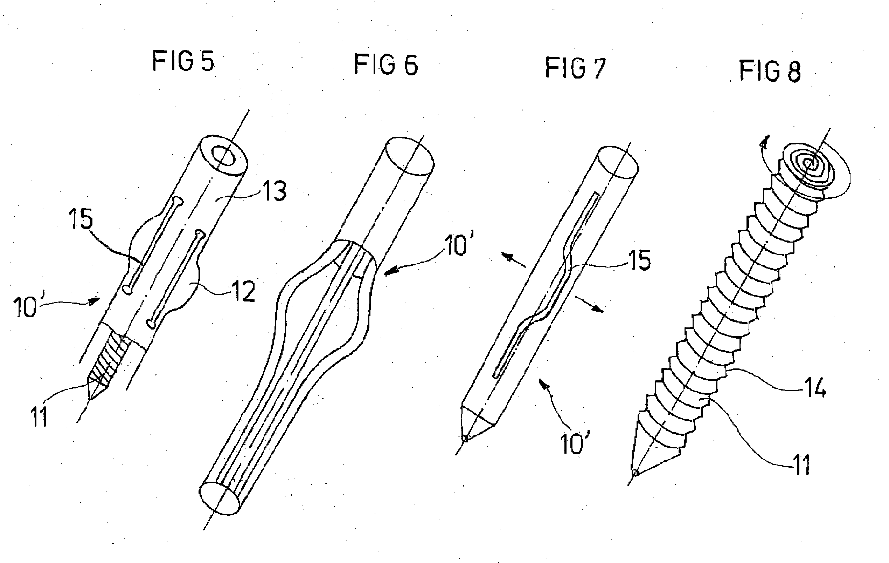

Die Figuren 5 bis 8 zeigen ein weiteres Ausführungsbeispiel einer Hülse 10', welche ohne eine Schraube eingesetzt werden kann. Die Hülse 10' ist aus einem Memory-Metall, insbesondere Nitinol, gefertigt, welches die Form insbesondere bei Erreichen der Körpertemperatur verändert. Die Hülse 10' weist ebenfalls in Längsrichtung verlaufende Schlitze 15 auf, zwischen welchen die Spreizelemente 16 gebildet werden. Nachdem die Hülse 10' in die Wirbelkörper 51, 52 eingesetzt wurde und die Körpertemperatur erreicht wurde, bewegen sich der vordere Abschnitt 11 und der hintere Abschnitt 13 relativ gegeneinander aufeinander zu, sodass in dem mittleren Bereich 12 die Spreizelemente 16 aufgespreizt werden (vgl. Figur 6). Dabei ist die Hülse 10' über das Außengewinde 14 des vorderen Bereichs 11 in dem Wirbelkörper 52 stabilisiert.

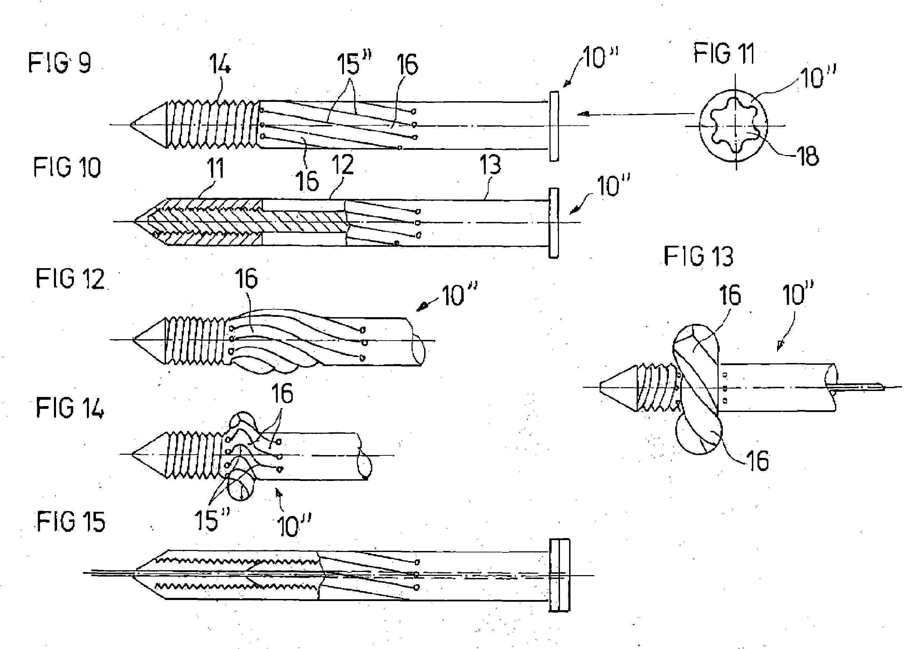

In den Figuren 9 bis 15 ist ein drittes Ausführungsbeispiel einer Hülse 10" dargestellt. Die Hülse 10" weist Schlitze 15" auf, welche sich in Längsrichtung erstrecken, jedoch gegen die Längsrichtung geneigt verlaufen. Werden der vordere Bereich 11 und der hintere Bereich 13 aufeinander zu bewegt, und dabei insbesondere gegeneinander verdreht, spreizen sich die zwischen den Schlitzen 15'' gebildeten Spreizelemente 16 auf, wobei sie insbesondere bei Verdrehen des vorderen Abschnitts 11 gegen den hinteren Abschnitt 13 nahezu aneinander anliegen bleiben und einen umlaufenden Wulst bilden (vgl. Figur 13).

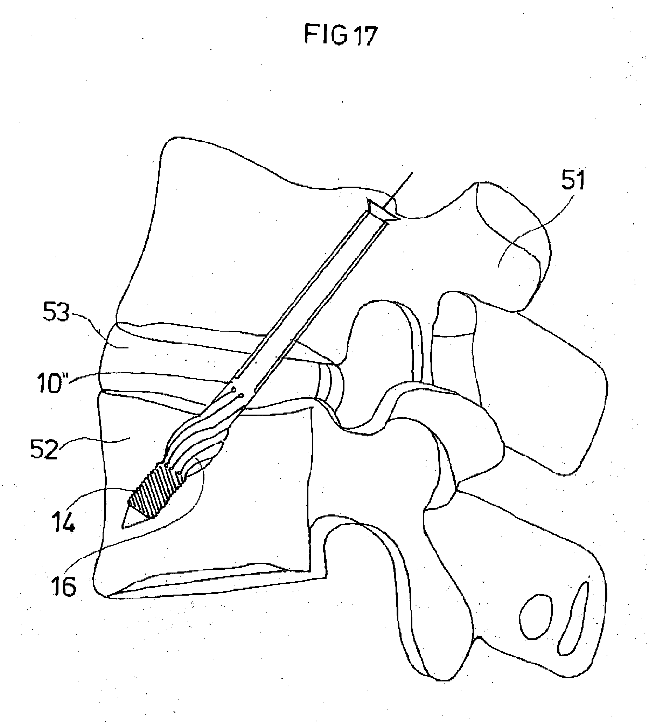

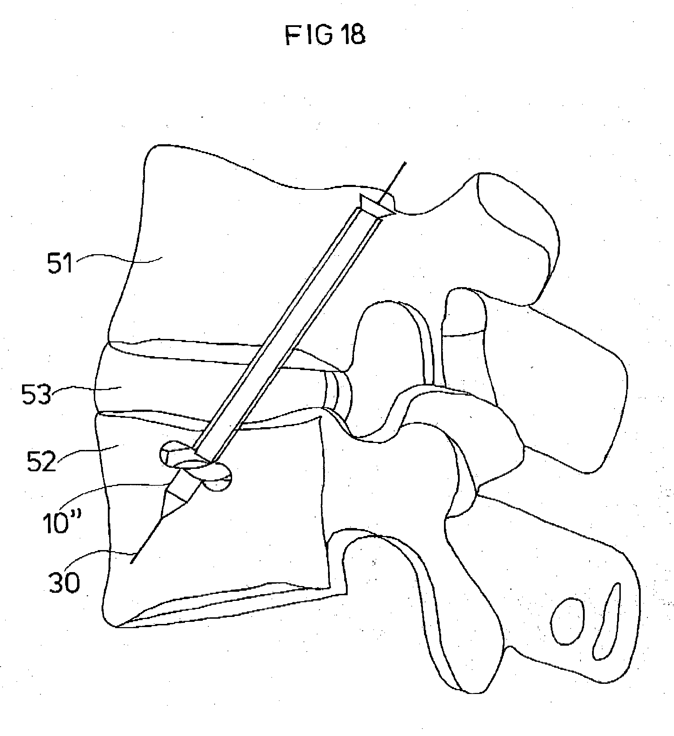

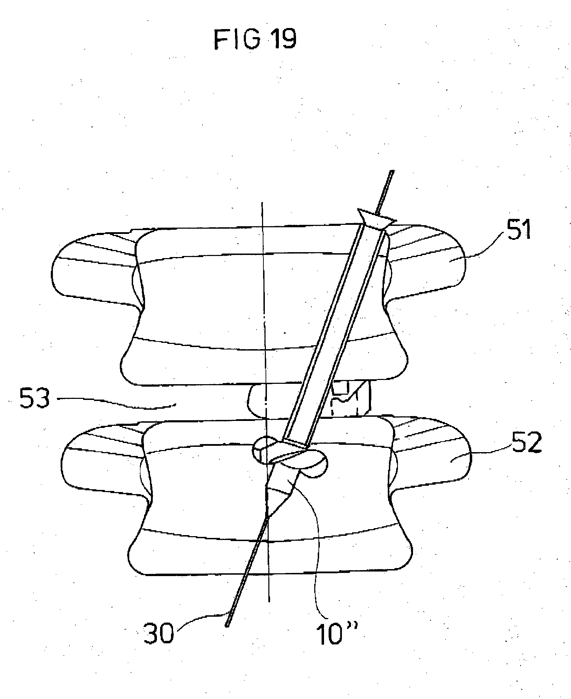

In den Figuren 17 bis 19 ist dargestellt, wie die Hülse 10' in den benachbarten Wirbelkörpern 51, 52 zu liegen kommt. Die Hülse 10' wird durch den oberen Wirbelkörper 51 eingeführt, durchsetzt den Zwischenwirbelraum 53 und wird soweit eingeführt, dass der vordere Bereich 11 und der mittlere Bereich 12 in dem unteren Wirbelkörper 52 zu liegen kommen. Die Spreizelemente 16 stabilisieren nach Aufspreizen der Hülse 10' den unteren Wirbelkörper 52 (vgl. Figuren 18 und 19).

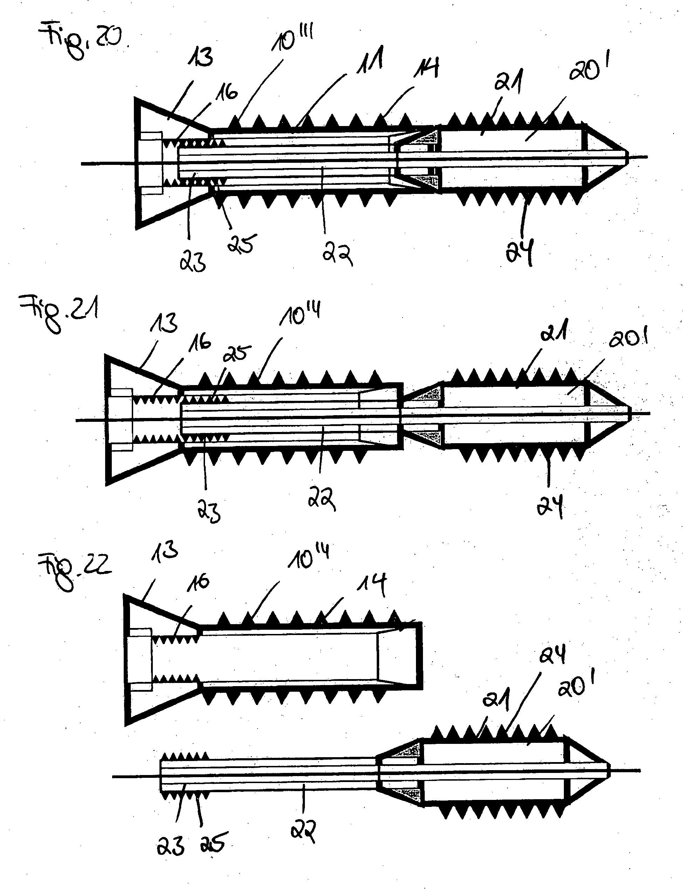

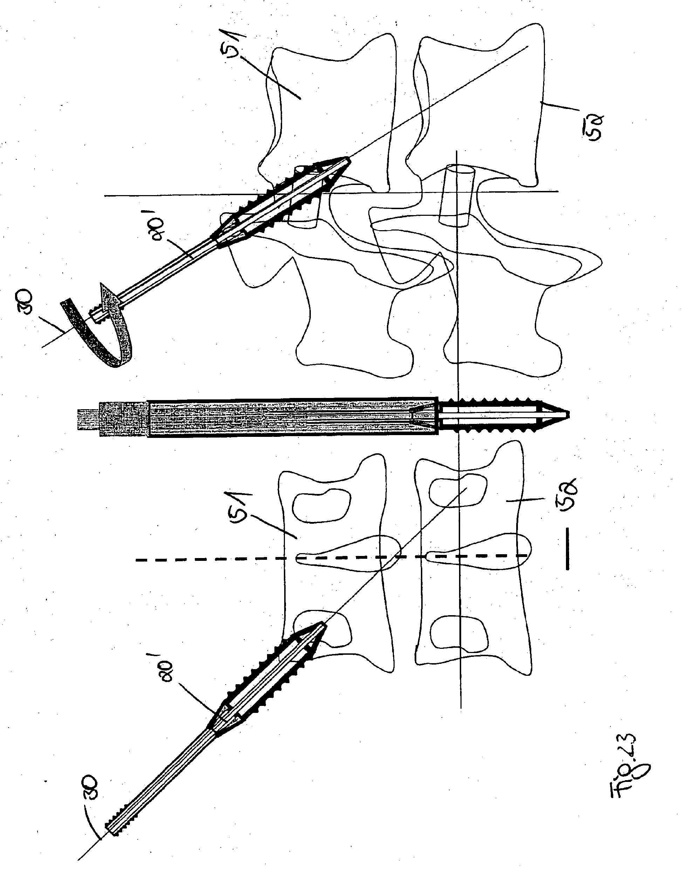

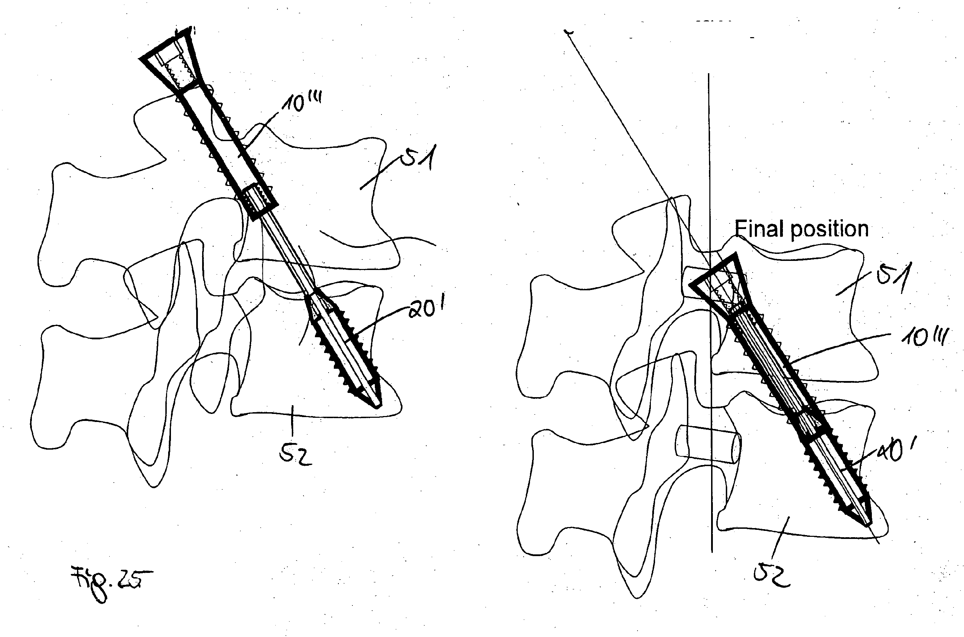

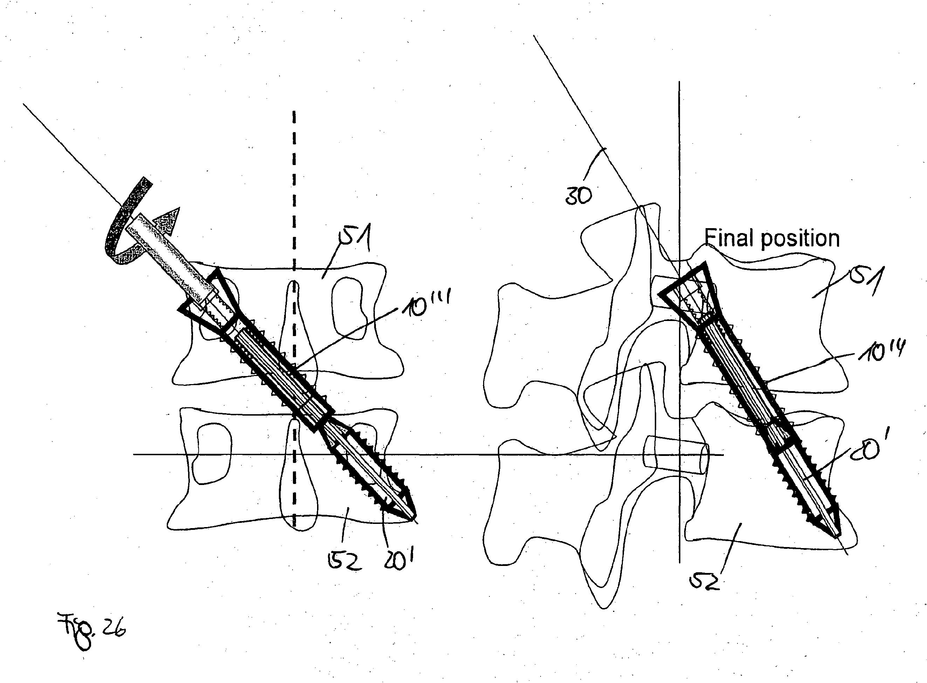

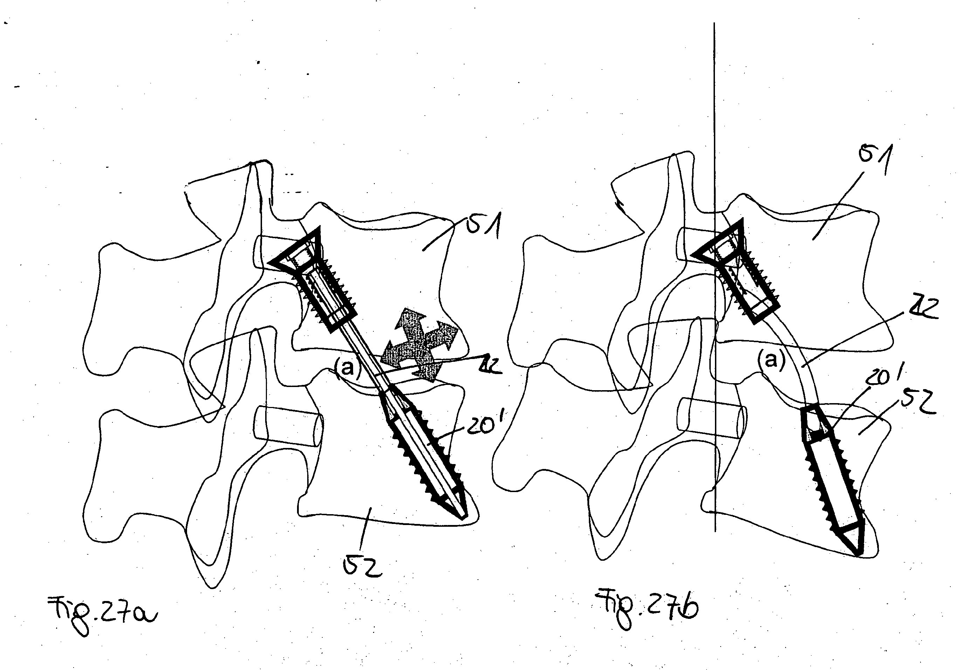

In den Figuren 20 bis 22 ist ein weiteres Ausführungsbeispiel einer Hülse 10''' mit einem weiteren Ausführungsbeispiel einer Schraube 20' dargestellt. Die Figuren 23 bis 27a, b zeigen, wie die die Hülse 10''' und die Schraube 20' in zwei benachbarte Wirbelkörper 51, 52 eingebracht werden.

Die Figuren 20 bis 22 zeigen verschiedene Ansichten eines ersten Ausführungsbeispiels einer Hülse 10''' mit einem vorderen Abschnitt 11 und einem sich daran anschließenden hinteren Bereich 13. Der vordere Bereich 11 trägt ein Außengewinde 14. In dem hinteren Abschnitt 13 ist ein Innengewinde 16 mit einer ersten Steigung angeordnet. Zur besseren Stabilisierung kann der hintere Abschnitt 13 konisch ausgebildet sein.

Die Schraube 20' weist einen vorderen Abschnitt 21, einen daran anschließenden mittleren Bereich 22 und einen daran anschließenden hinteren Abschnitt 23 auf. In dem vorderen Abschnitt 21 der Schraube 20 ist ein erstes Außengewinde 24 angeordnet, während in dem hinteren Abschnitt 23 ein zweites Au-βengewinde 25 angeordnet ist. Das erste Außengewinde 24 weist dabei eine dritte Steigung auf, während das zweite Außengewinde 25 eine vierte Steigung aufweist. Die vierte Steigung des zweiten Außengewindes 25 entspricht jedoch insbesondere der ersten Steigung des ersten Innengewindes 16 der Hülse 10. Die dritte Steigung und die vierte Steigung können dabei unterschiedlich gewählt werden.

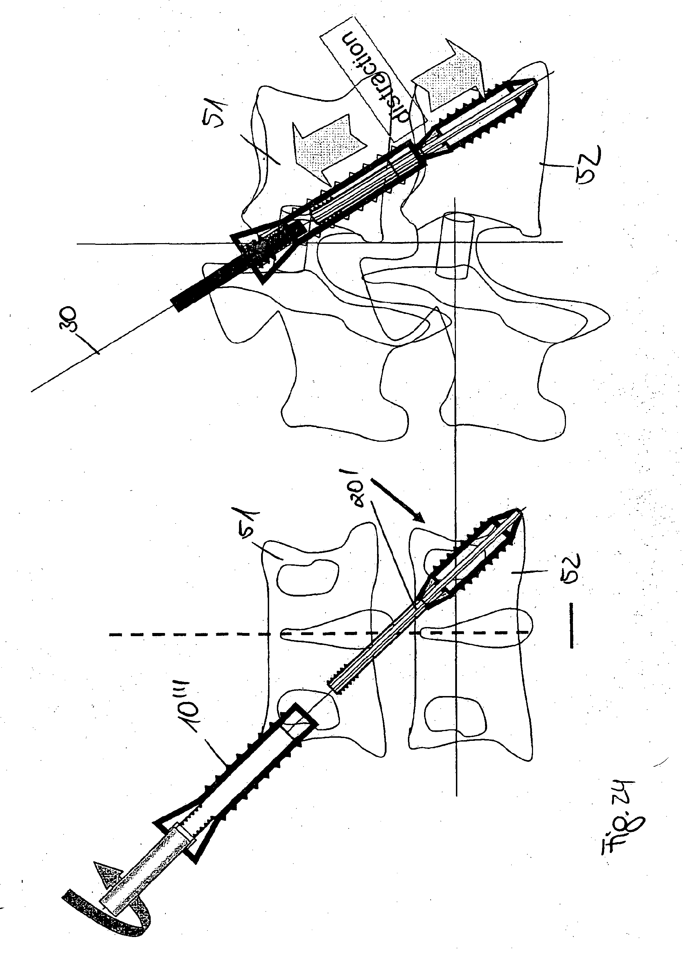

Wie in den Figuren 23 bis 27a, b erkennbar, wird zunächst die Schraube 20', insbesondere entlang eines Führungsdrahts 30, in die Wirbelkörper 51, 52 eingeführt, wobei die Schraube 20' durch den oberen Wirbelkörper 51 bis in den unteren Wirbelkörper 52 eingedreht wird, wobei die Schraube 20' den Zwischenwirbelraum 53 durchsetzt. Der mittlere Bereich 12 kommt im Zwischenwirbelraum 53 zu liegen (vgl. Figuren 23 und 24). Anschließend wird die Hülse 10''' auf die Schraube 20' gedreht (vgl. Figuren 24 bis 27a, b). Dabei greift das zweite Außengewinde 25 der Schraube 20' in das Innengewinde 16 der Hülse 10''' ein. Die Hülse 10''' kann insbesondere so weit eingedreht werden, dass ein Anschlag 26 der Schraube 20' gegen den distalen Rand der Hülse 10''' gezogen wird und anschließend die Wirbelkörper 51, 52 distrahiert werden (vgl. Figur 24, 25 und 26). Alternativ kann auch die Hülse 10''' nur derart weit aufgeschraubt werden, dass sie im superioren Wirbelkörper 51 verbleibt und lediglich der mittlere Abschnitt 22 der Schraube 20' den Zwischenwirbelraum 53 durchsetzt (vgl. Figur 27a, b).

In einer Ausführungsform ist der mittlere Abschnitt 22 der Schraube 20' elastisch ausgebildet. Wird die Hülse 10''' nur derart weit aufgeschraubt, dass sie im superioren Wirbelkörper 51 verbleibt und lediglich der mittlere Abschnitt 22 der Schraube 20' den Zwischenwirbelraum 53 durchsetzt, besteht die Möglichkeit, die Wirbelkörper 51, 52 gegeneinander zu kippen, zu drehen und relativ zueinander zu bewegen, so dass auf diese Weise eine Bandscheibe simuliert werden kann.

### Bezugszeichenliste

- 10, 10', 10'': Hülse
- 11: Vorderer Abschnitt
- 12: Mittlerer Abschnitt
- 13: Hinterer Abschnitt
- 14: Außengewinde
- 15, 15', 15'': Schlitz
- 16: Spreizelement
- 17: Sollknickstelle
- 18: Kontur

- 20: Schraube
- 20a: Schaft
- 20b: Kopf
- 21: Vorderer Abschnitt
- 22: Mittlerer Abschnitt
- 23: Hinterer Abschnitt
- 24: Erstes Außengewinde
- 25: Zweites Außengewinde
- 26: Anschlag

- 30: Führungsdraht

- 40: Eindrehinstrument

- 51: Wirbelkörper
- 52: Wirbelkörper
- 53: Zwischenwirbelraum

## Patentansprüche

1. Vorrichtung zum Stabilisieren einer Wirbelsäule mit einer Hülse (10, 10', 10'') zum Einbringen in einen Wirbelkörper (51, 52), welche in einem Abschnitt (11) ein Außengewinde (14) aufweist, und mit einer Schraube (20),
**dadurch gekennzeichnet, dass** die Hülse (10, 10', 10'') ein erstes Innengewinde mit einer ersten Steigung aufweist, in welches die Schraube (20) eindrehbar ist, dass die Hülse (10, 10', 10'') einen vorderen Abschnitt (11), einen mittleren Abschnitt (12) und einen hinteren Abschnitt (13) aufweist, wobei der vordere Abschnitt (11) das Außengewinde (14) aufweist und in dem mittleren Abschnitt (12) wenigstens ein Spreizelement (16), vorzugsweise mehrere Spreizelemente (16), angeordnet ist, dass in dem mittleren Abschnitt (12) der Hülse (10, 10', 10'') Öffnungen in Form von in Längsrichtung der Hülse (10, 10', 10'') angeordneten Schlitzen (15, 15', 15''), angeordnet sind, zwischen welchen die Spreizelemente (16) gebildet sind und dass entweder die Hülsen (10, 10', 10'') das erste Innengewinde in dem vorderen Abschnitt (11) aufweist und
dass die Schraube (20) einen vorderen Abschnitt (21) mit einem ersten Außengewinde (24) mit einer dritten Steigung und einen hinteren Abschnitt (23) mit einem zweiten Außengewinde (25) mit einer vierten Steigung aufweist, wobei die dritte Steigung der ersten Steigung entspricht und die vierte Steigung von der dritten Steigung verschieden ist
oder
dass die Hülse (10''') das erste Innengewinde in einem hinteren Abschnitt (13) aufweist und dass die Schraube (20') einen vorderen Abschnitt (21) mit einem ersten Außengewinde (24) mit einer dritten Steigung und einen hinteren Abschnitt (23) mit einem zweiten Außengewinde (25) mit einer vierten Steigung aufweist, wobei die vierte Steigung der ersten Steigung entspricht.

2. Vorrichtung nach Anspruch 1,
**dadurch gekennzeichnet, dass** die Spreizelemente (16) in Umfangsrichtung der Hülse (10, 10', 10'') unterschiedlich ausgebildet sind.

3. Vorrichtung nach einem der Anspruche 1 oder 2,
**dadurch gekennzeichnet, dass** die Spreizelemente (16) Sollknickstellen (17) aufweisen.

4. Vorrichtung nach einem der vorhergehenden Ansprüche
**dadurch gekennzeichnet, dass** die Schraube (20) an ihrem Schraubenschaft (20a) einen Anschlag (26) aufweist, welcher insbesondere konisch ausgebildet ist.

5. Vorrichtung nach einem der vorhergehenden Ansprüche,
**dadurch gekennzeichnet, dass** die Hülse (10') zumindest in dem mittleren Abschnitt (12) aus einer Memory-Legierung, insbesondere aus Nitinol, gefertigt ist.

6. Vorrichtung nach einem der vorhergehenden Ansprüche,
**dadurch gekennzeichnet, dass** die Hülse (10, 10' , 10'') und die Schraube (20) kannuliert ausgebildet sind.

7. Vorrichtung zum Stabilisieren einer Wirbelsäule mit einer Hülse (10"') zum Einbringen in einen Wirbelkörper (51, 52), welche in einem Abschnitt (11) ein Außengewinde (14) aufweist, und mit einer Schraube (20'),
**dadurch gekennzeichnet, dass** die Hülse (10"') ein erstes Innengewinde mit einer ersten Steigung aufweist, in welches die Schraube (20') eindrehbar ist, dass die Hülse (10"') einen vorderen Abschnitt (11), einen mittleren Abschnitt (12) und einen hinteren Abschnitt (13) aufweist, wobei der vordere Abschnitt (11) das Außengewinde (14) aufweist und in dem mittleren Abschnitt (12) wenigstens ein Spreizelement (16), vorzugsweise mehrere Spreizelemente (16), angeordnet ist, dass in dem mittleren Abschnitt (12) der Hülse (10"') Öffnungen in Form von in Längsrichtung der Hülse ( 10"') angeordneten Schlitzen, angeordnet sind, zwischen welchen die Spreizelemente (16) gebildet sind und dass die Hülse (10''') das erste Innengewinde in einem hinteren Abschnitt (13) aufweist und dass die Schraube (20') einen vorderen Abschnitt (21) mit einem ersten Außengewinde (24) mit einer dritten Steigung und einen hinteren Abschnitt (22) mit einem zweiten Außengewinde (25) mit einer vierten Steigung aufweist, wobei die vierte Steigung der ersten Steigung entspricht und dass die Schraube (20') in einem Bereich zwischen dem vorderen Abschnitt (21) und dem hinteren Abschnitt (22) elastisch ausgebildet ist.

## Claims

1. Device for stabilising a spinal column with a sleeve (10, 10', 10") for introduction into a vertebral body (51, 52), having an external thread (14) in one portion (11), and with a screw (20), **characterised in that** the sleeve (10, 10', 10") has a first internal thread with a first pitch into which the screw (20) can be screwed, that the sleeve (10, 10', 10") has a front portion (11), a central portion (12) and a rear portion (13), wherein the front portion (11) has the external thread (14) and at least one expansion element (16), preferably a plurality of expansion elements (16), is disposed in the central portion (12), that openings in the form of slots (15, 15', 15") disposed in the longitudinal direction of the sleeve (10, 10', 10") are disposed in the central portion (12) of the sleeve (10, 10', 10"), the expansion elements (16) being formed between the said slots, and that either the sleeve (10, 10', 10") has the first internal thread in the front portion (11) and that the screw (20) has a front portion (21) with a first external thread (24) with a third pitch and a rear portion (23) with a second external thread (25) with a fourth pitch, wherein the third pitch corresponds to the first pitch and the fourth pitch differs from the third pitch, or that the sleeve (10'") has the first internal thread in a rear portion (13) and that the screw (20') has a front portion (21) with a first external thread (24) with a third pitch and a rear portion (23) with a second external thread (25) with a fourth pitch, wherein in particular the fourth pitch corresponds to the first pitch.

2. Device according to claim 1, **characterised in that** the expansion elements (16) are constructed differently in the circumferential direction of the sleeve (10, 10', 10").

3. Device according to one of claims 1 or 2, **characterised in that** the expansion elements (16) have predetermined bending points (17).

4. Device according to one of the preceding claims, **characterised in that** the screw (20) has on its shank (20a) a stop (26) which is in particular conical.

5. Device according to one of the preceding claims, **characterised in that** the sleeve (10') is manufactured, at least in the central portion (12), from a memory alloy, in particular from nitinol.

6. Device according to one of the preceding claims, **characterised in that** the sleeve (10, 10', 10") and the screw (20) are cannulated.

7. Device for stabilising a spinal column with a sleeve (10"') for introduction into a vertebral body (51, 52), having an external thread (14) in one portion (11), and with a screw (20'), **characterised in that** the sleeve (10"') has a first internal thread with a first pitch into which the screw (20') can be screwed, that the sleeve (10"') has a front portion (11), a central portion (12) and a rear portion (13), wherein the front portion (11) has the external thread (14) and at least one expansion element (16), preferably a plurality of expansion elements (16), is disposed in the central portion (12), that openings in the form of slots disposed in the longitudinal direction of the sleeve (10"') are disposed in the central portion (12) of the sleeve (10'"), the expansion elements (16) being formed between the said slots, that the sleeve (10"') has the first internal thread in a rear portion (13) and that the screw (20') has a front portion (21) with a first external thread (24) with a third pitch and a rear portion (22) with a second external thread (25) with a fourth pitch, wherein the fourth pitch corresponds to the first pitch, and that the screw (20') is constructed resiliently in a region between the front portion (21) and the rear portion (22).

## Revendications

1. Dispositif de stabilisation d'une colonne vertébrale comprenant un manchon (10, 10', 10") destiné à être inséré dans un corps vertébral (51, 52) et comportant un filetage externe (14) dans un segment (11) ainsi qu'une vis (20),
**caractérisé en ce que**
le manchon (10, 10', 10") comporte un premier taraudage ayant un premier pas dans lequel la vis (20) peut être introduite par rotation, le manchon (10, 10', 10") comporte un segment avant (11), un segment médian (12) et un segment arrière (13), le segment avant (11) étant équipé du filetage externe (14) et, dans le segment médian (12) étant positionné au moins un élément extensible (16) de préférence plusieurs éléments extensibles (16), dans le segment médian (12) du manchon (10, 10', 10") sont positionnées des ouvertures réalisées sous la forme de fentes (15, 15', 15") s'étendant dans la direction longitudinale du manchon (10, 10', 10") et entre lesquelles sont formés les éléments extensibles (16), et, soit le manchon (10, 10', 10") comporte le premier taraudage dans le segment avant (11) et la vis (20) comporte un segment avant (21) avec un premier filetage externe (24) ayant un troisième pas et un segment arrière (23) ayant un second filetage externe (25) avec un quatrième pas, le troisième pas correspondant au premier pas et le quatrième pas étant différent du troisième pas, soit
le manchon (10'") comporte le premier taraudage dans un segment arrière (13) et la vis (20') comporte un segment avant (21) ayant un premier filetage externe (24) avec un troisième pas et un segment arrière (23) ayant un second filetage externe (25) avec un quatrième pas, le quatrième pas correspondant au premier pas.

2. Dispositif conforme à la revendication 1,
**caractérisé en ce que**
les éléments extensibles (16) sont réalisés différemment dans la direction périphérique du manchon (10, 10', 10").

3. Dispositif conforme à l'une des revendications 1 et 2,
**caractérisé en ce que**
les éléments extensibles (16) comprennent des points d'articulation de consigne (17).

4. Dispositif conforme à l'une des revendications précédentes,
**caractérisé en ce que**
la vis (20) comporte sur sa tige (20a) une butée (26) qui est en particulier de forme conique.

5. Dispositif conforme à l'une des revendications précédentes,
**caractérisé en ce que**
le manchon (10') est réalisé au moins dans son segment médian (12) en un alliage à mémoire de forme, en particulier en nitinol.

6. Dispositif conforme à l'une des revendications précédentes,
**caractérisé en ce que**
le manchon (10, 10', 10") et la vis (20) sont cannelés.

7. Dispositif de stabilisation d'une colonne vertébrale comportant un manchon (10"') destiné à être inséré dans un corps vertébral (51, 52) qui comporte un filetage externe (14) dans un segment (11) ainsi qu'une vis (20'), **caractérisé en ce que** le manchon (10"') comporte un premier taraudage ayant un premier pas dans lequel la vis (20') peut être introduite par rotation, le manchon (10'") comporte un segment avant (11), un segment médian (12) et un segment arrière (13), le segment avant (11) comportant le filetage externe (14) et dans le segment médian (12) étant positionné au moins un élément extensible (16) de préférence plusieurs éléments extensibles (16), dans le segment médian (12) du manchon (10"') sont positionnées des ouvertures réalisées sous la forme de fentes s'étendant dans la direction longitudinale du manchon (10"') entre lesquelles sont formés les éléments extensibles (16), le manchon (10'") comprend le premier taraudage dans son segment arrière (13) et la vis (20') comprend un segment avant (21) ayant un premier filetage externe (24) avec un troisième pas et un segment arrière (22) ayant un second filetage externe (25) avec un quatrième pas, le quatrième pas correspondant au premier pas, et, la vis (20') est réalisée élastiquement dans une zone comprise entre le segment avant (21) et le segment arrière (22).
